# EUROPEAN PATENT APPLICATION

(11) **EP 4 070 746 A1**
(43) Date of publication of application: **12.10.2022**
(21) Application number: 20896701.8
(22) Date of filing: 27.11.2020
(51) Int. Cl.: A61B 17/42

(54) **UNIT-TYPE HYSTEROSCOPE INSTRUMENT OPERATION PUSHING DEVICE**

(30) Priority: 06.12.2019 CN 201922179867 U; 15.05.2020 CN 202020819675 U; 15.05.2020 CN 202010414107
(71) Applicant: Zhang, Chongyi, Xi'an, Shaanxi 710077 (CN)
(72) Inventor: Zhang, Chongyi, Xi'an, Shaanxi 710077 (CN)
(74) Representative: Ehrner & Delmar Patentbyrå AB
(86) International application number: PCT/CN2020/132420
(87) International publication number: WO 2021/109942

(57) **Abstract**

The present invention disclosed a single-piece hysteroscope instrument operation propeller, which includes a propelling body that can be attached to the hysteroscope body, the instrument can smoothly pass through the blind areas such as the vagina and cervical canal along the hysteroscope body that has already entered with the help of the groove on the propelling body, and can be operated continuously and flexibly after being sent into the uterus. The present invention is beneficial for doctors to quickly, accurately and non-invasively send the treatment instrument into the uterus, which not only reduces the treatment cost, but also achieves a good treatment effect.

## Description

### TECHNICAL FIELD

The present invention relates to a hysteroscope surgical treatment system, and more particularly to a device attached to a hysteroscope body to assist instruments to enter the uterine cavity.

### BACKGROUND

In the process of hysteroscopic treatment, the commonly used resectoscope, therapeutic mirror and planing system have in common that the inspection mirror body and multiple therapeutic instruments are wrapped together, resulting in a relatively thick system, which often needs to dilate the cervix to more than 8mm. Moreover, the instruments used for treatment operations are generally fine in structure, often expensive, complicated and time-consuming to operate, which makes it difficult to perform hysteroscopic surgery using these systems in outpatient clinics.

In the process of hysteroscopy, even if polyps, embryo residues, or intrauterine adhesions are found in the uterine cavity, it is not easy due to scissors, forceps and other therapeutic instruments alone are not easy to pass through blind spots such as vagina and cervical canal, which requires a higher skill for the doctors to further carry out hysteroscopic treatment. In addition, due to the front ends of the scissors and the forceps are sharp, and is easy to scratch the mucosa of the vagina and cervical canal, such that the widespread outpatient hysteroscopic surgery has multiple limiting factors.

Chinese patent CN201379632Y discloses a uterine cavity surgical instrument, in which the uterine forceps and the hysteroscope can be moved synchronously and can be operated independently at the same time to form a system. It can ensure that the entry and operation of the instruments are under the monitoring of the hysteroscope, but the hysteroscope needs to be returned when the instruments need to be replaced, which leads to a long time for the entire treatment process, and the limited operating range of the instruments leads to the view of the hysteroscope frequently replacing, the continuity of the operation is reduced and the treatment effect is affected.

### TECHNICAL PROBLEM

An object of the present invention is to provide a single-piece hysteroscope instrument operation propeller, the structure is simple and is easy to be operated.

### SUMMARY

In order to achieve above object, the present invention adopts the technical solution as following:
The propeller includes a cylindrical propelling body, one side surface of the cylindrical propelling body is provided with an engagement groove configured fitting with a hysteroscope body, and the other side surface of the cylindrical propelling body is provided with a guiding groove (the transverse section is arc-shaped) extending along the hysteroscope body.

In one embodiment, a transverse section (referring to the surface of the cylindrical propelling body contacting with the hysteroscope body) of the engagement groove is arc-shaped (a radian of the engagement groove is greater than π, such that the cylindrical propelling body can be firmly attached to the hysteroscope body to prevent the cylindrical propelling body from falling off).

In one embodiment, a length of the cylindrical propelling body is less than a length of the hysteroscope body.

In one embodiment, the cylindrical propelling body is provided with a push-pull handle (convenient for holding and operating) configured for sliding the hysteroscope body relative to the engagement groove.

In one embodiment, the push-pull handle is arranged at an end surface of the cylindrical propelling body, and the other end surface of the cylindrical propelling body is close to (relative to the end surface where the push-pull handle located) a front end (the collecting end of the checking image) of the hysteroscope body.

In one embodiment, a surface portion of the cylindrical propelling body located between an opening of the engagement groove and the guiding convex strip is protruded outwardly (which constitutes the remaining surface of the cylindrical propelling body except for the engagement groove, the guiding convex strip, and the end surface), and the protruded surface portion is divided into two sections symmetrically distributed along a longitudinal section (taking an axial direction of the hysteroscope body as the longitudinal direction) of the cylindrical propelling body, and a transverse section of an outwardly protruded surface of each section is arc-shaped.

In one embodiment, the propeller can be used with a speculum or without a speculum; for example, the hysteroscope body, the cylindrical propelling body and the instruments can be passed through the speculum into the uterine cavity, and the colposcopy technique can also be used without using a speculum, directly into the hysteroscope body, after the hysteroscope body enters the uterine cavity, and then use the cylindrical propelling body to send the instrument into the uterine cavity.

The method for operating the propeller above includes following steps:
1) combining the cylindrical propelling body with the hysteroscope body through the engagement groove;
2) sending the hysteroscope body into a uterine cavity; and
3) adjusting a position of the cylindrical propelling body along the hysteroscope body under a hysteroscope view, such that a surgical instrument is able to enter the uterine cavity along the guiding groove.

### BENEFICAL EFFECTS

In the present invention, by using the engagement groove of the cylindrical propelling body, the guiding groove can be introduced along the hysteroscope body and fixed in the channel entering the uterine cavity, such that the instrument can be quickly, accurately and non-invasively sent into the uterine cavity without hysteroscope monitoring, and once the front end of the instrument enters the hysteroscope monitoring area, the doctor can continuously complete a surgical operation of lesions (polyps, embryo residues, or intrauterine adhesions present in the uterine cavity) at different angles and depths (the operating range is larger and the flexibility is larger) within a certain field of view, such that the treatment process is simple and does not require to dilate the cervix or the size that need to dilate the cervix is reduced, the operation process is also safer, the treatment cost and time are reduced, and the pain degree of treatment is reduced, and the corresponding operation can be completed in the outpatient clinic. In addition, the popularization and application of the present invention can also reduce the cost of the instrument (the instrument with a simpler structure can be used, and the instrument does not need complicated and fine structural design).

Further, in the present invention, the engagement groove, the convex surface between the guiding groove and the engagement groove are all curved surfaces without edges and corners, when combining the using of the guiding groove, the entering process of the propeller and the instrument along the hysteroscope body is relatively stable and avoids damage to normal tissues such as the uterine cavity.

### BRIEF DESCRIPTION OF THE DRAWINGS

Fig. 1 is a front view of a hysteroscopic instrument operation propeller in an embodiment 1;
Fig. 2 is the bottom view of a hysteroscopic instrument operation propeller in an embodiment 1;
Fig. 3 is a schematic view of a transverse section (A-A) of a hysteroscopic instrument operation propeller in an embodiment 1;
Fig. 4 is a perspective view of the hysteroscopic instrument operation propeller in an embodiment 2;

In the figures:
1 hysteroscope body; 1-1 rear end of the hysteroscope body; 1-2 front end of the hysteroscope body; 2 propelling body; 2-1 handle; 2-2 groove.

### DETAILED DESCRIPTION

The present invention will be described in further detail below with reference to the accompanying drawings and embodiments.

### Embodiment 1:

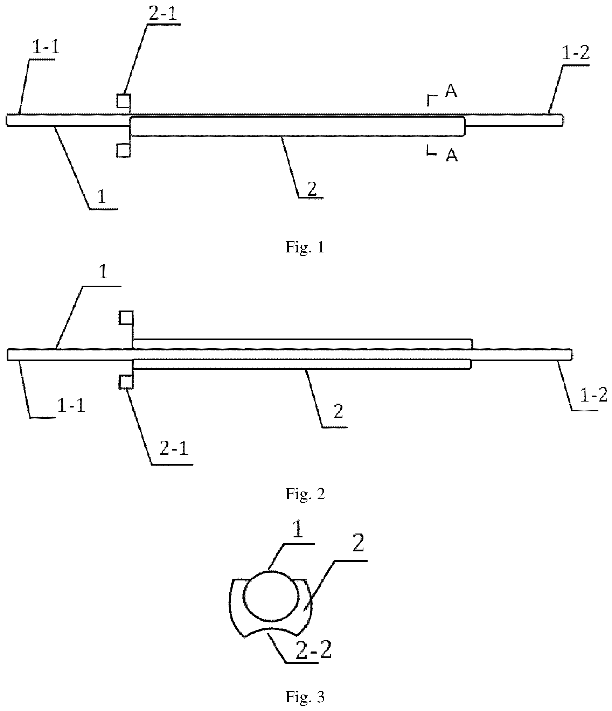

Referring to Fig. 1, Fig. 2 and Fig. 3, the hysteroscopic instrument operation propeller according to the present invention includes a propelling body 2, and one side surface of the propelling body 2 is a concave large semi-cylindrical surface, forming the engagement groove of the propelling body 2, the propelling body 2 can be reliably attached to the hysteroscope body 1 through the engagement groove, and the other side surface of the propelling body 2 (the back of the engagement groove) is provided with a groove 2-2 which is convenient for the instrument with a sharp front end to be sent into the uterine cavity along the hysteroscope body 1 (the front end of the instrument moves along the groove); the surface portion of the propelling body 2 between the opening of the engagement groove and the corresponding side of the groove 2-2 is an outwardly convex arc-shaped, which can reduce the interference with the surrounding tissue. The surrounding tissue can also be separated from the groove 2-2 to a certain extent, so as to avoid the hooking between the instrument and the surrounding tissue at the position of the groove. The length of the propelling body 2 is about 100 mm (the length of the hysteroscope body is about 200mm), and which can be made of stainless steel. In order to adjust the distance between the front end of the propelling body 2 and the front end of the hysteroscope body (for example, after the propelling body 2 is observed in the hysteroscope monitoring area) after the propelling body 2 is engaged on the hysteroscope body 1, a square handle 2-1 is provided on a rear end of the propelling body 2. The front end of the propelling body 2 can be processed into an arc-shaped to facilitate the movement of the propelling body 2 (for example, to reduce hooking and dragging with surrounding tissues), and which can also reduce the shielding of the hysteroscope monitoring area (especially when the front end of the propelling body 2 approaches the front end 1-2 of the hysteroscope body or overlaps with the front end 1-2 of the hysteroscope body), such that the groove 2-2 can be positioned at a suitable position, so as to fully play a guiding role in the movement of the instrument.

During hysteroscopic surgery, the propelling body 2 is firstly installed (pressed or inserted) on the rear end 1-1 of the hysteroscope body, then the hysteroscope body 1 is sent into the uterus. During the hysteroscopy process, when polyps, embryo residues, or intrauterine adhesions, etc. are found in the uterine cavity, the instrument can be sent for treatment. At this time, according to the width of the internal orifice of the cervical canal, the front end 1-2 of the hysteroscope body is kept at the uterine cavity or the internal orifice of the cervical canal, and the front end of the propelling body 2 is pushed into the front end 1-2 of the hysteroscope body using the handle 2-1 and stop until the front end of the propelling body 2 can be seen on the display of the hysteroscope, and the propelling body 2 can be pushed or pulled slightly to adjust the position of the front end of the propelling body 2 (to ensure that the field of view of the hysteroscope monitoring area on the display is always good before and after the instrument enters along the groove 2-2), the head end of the scissors or forceps of the laparoscope is put into (for example, engaged into) the rear end of the groove 2-2, and then the scissor or plier is sent to the front end of the propelling body 2 along the groove 2-2 and slide out, the forceps or scissors can be seen entering the uterus on the display of the hysteroscope, and then normal treatment operations are performed. When it is necessary to replace the instrument for the treatment operation, withdraw the instrument used in the current operation, and then repeat the above method of sending the instrument, and then the new treatment operation can be continued.

Since polyps, embryo residues, or intrauterine adhesions are found in the uterine cavity during the hysteroscopy, it was necessary to enter the treatment instruments. By using the above propeller, when the instruments pass through the blind areas of the vagina and cervical canal, which can be smoothly sent into the uterus along the groove 2-2 that has already entered and positioned on the surface of the hysteroscope body 1, therefore, the interval time between treatment operations can be shortened, and since the device integrating the hysteroscope body 1 and the instrument is no longer required, the cervix is required to be about 6mm, so that some conventional treatments basically do not need to dilate of the cervix, which can reduce the intraoperative pain of the patient and avoid hospitalization. Furthermore, the operation of the instrument is less restricted, and it can be operated continuously and flexibly under the hysteroscopic field of view provided by the display. In addition, it can also meet the objective requirements for the durability and easy availability of the treatment instrument, and selecting to use the treatment instrument with the structure being simpler and the size being larger (which makes the instrument easier to handle and less prone to damage).

### Embodiment 2:

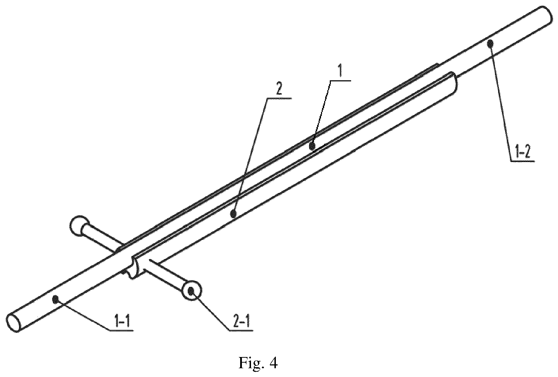

Referring to Fig. 4, the difference from the embodiment 1 is that the handle 2-1 at the rear end of the propelling body 2 is circular, which is easier to be held and operated.

In a word, the hysteroscopic instrument operation propeller of the present invention is beneficial for doctors to quickly, accurately and non-invasively send the treatment instrument into the uterus, and the treatment process can be completed in the outpatient operating room, which not only reduces the treatment cost, but also achieves a good treatment effect.

## Claims

1. A single-piece hysteroscopic instrument operation propeller, comprising:
a cylindrical propelling body (2), one side surface of the cylindrical propelling body (2) being provided with an engagement groove configured fitting with a hysteroscope body (1), and the other side surface of the cylindrical propelling body (2) being provided with a guiding groove (2-2) extending along the hysteroscope body (1).

2. The single-piece hysteroscopic instrument operation propeller according to claim 1, wherein a transverse section of the engagement groove is arc-shaped.

3. The single-piece hysteroscopic instrument operation propeller according to claim 2, wherein a radian of the engagement groove is greater than π.

4. The single-piece hysteroscopic instrument operation propeller according to claim 1, wherein a length of the cylindrical propelling body (2) is less than a length of the hysteroscope body (1).

5. The single-piece hysteroscopic instrument operation propeller according to claim 1 or 4, wherein the cylindrical propelling body (2) is provided with a push-pull handle (2-1) configured for sliding the hysteroscope body (1) relative to the engagement groove.

6. The single-piece hysteroscopic instrument operation propeller according to claim 5, wherein the push-pull handle (2-1) is arranged at an end surface of the cylindrical propelling body (2), and the other end surface of the cylindrical propelling body (2) is close to a front end of the hysteroscope body (1).

7. The single-piece hysteroscopic instrument operation propeller according to claim 1, wherein a surface portion of the cylindrical propelling body (2) located between an opening of the engagement groove and an opening of the guiding groove (2-2) is protruded outwardly, and the protruded surface portion is divided into two sections symmetrically distributed along a longitudinal section of the cylindrical propelling body (2), and a transverse section of an outwardly protruded surface of each section is arc-shaped.

8. A method for operating the single-piece hysteroscopic instrument operation propeller according to claim 1, comprising following steps:
1) combining the cylindrical propelling body (2) with the hysteroscope body (1) through the engagement groove;
2) sending the hysteroscope body (1) into a uterine cavity; and
3) adjusting a position of the cylindrical propelling body (2) along the hysteroscope body (1) under a hysteroscope view, such that a surgical instrument is able to enter the uterine cavity along the guiding groove (2-2).
